# EUROPEAN PATENT APPLICATION

(11) **EP 3 536 242 A1**
(43) Date of publication of application: **11.09.2019**
(21) Application number: 19160466.9
(22) Date of filing: 04.03.2019
(51) Int. Cl.: A61B 6/00

(54) **APPARATUS FOR THE ANALYSIS OF SAMPLES PICKED UP BY BIOPSY WITH MOVABLE STABILISING ELEMENT**

(30) Priority: 09.03.2018 IT 201800003416
(71) Applicant: IMS Giotto S.p.A., 40037 Sasso Marconi (Bologna) (IT)
(72) Inventor: VECCHIO, Sara, 40033 Casalecchio di Reno (Bologna) (IT); VIGNOLI, Paolo, 40017 San Giovanni in Persiceto (Bologna) (IT)
(74) Representative: Falzoni, Davide Aldo

(57) **Abstract**

Described is an apparatus (1) for the analysis of samples picked up by biopsy from a part (M) of the body of a patient, by which a high precision of the bioptic picking up is obtained, even with changes in the shape and/or dimensional characteristics of the part (M) of the body being analysed.

## Description

This invention relates to an apparatus for the analysis of samples picked up by biopsy from a part of the body of a patient, by which a high precision of the bioptic picking up is obtained, even with changes in the shape and/or dimensional characteristics of the part of the body being analysed. Currently, in order to the perform the bioptic taking of lesions identified as suspicious in the body of the patient, one of the possibilities when the lesion is radiopaque is the use of an apparatus for the X-ray analysis of at least a part of the body of the patient, by which a research of the suspicious zone of the part of that body of the patient is performed. The suspicious zone means a zone in which there could be a lesion which is to be subsequently analysed. When the suspicious zone has been identified in the X-ray image, its position is determined by means of further X-ray images (pointing) and a step of taking one or more samples by means of a biopsy is performed, in order to sample a part or all of the suspicious zone, in order to subsequently analyse by cytological or histological examination. The apparatus includes a positioning surface for the positioning of the part of the body to be analysed and a biopsy probe to perform the bioptic sampling. The apparatus also includes a stabilising element configured to apply a stabilising action on the part of the body, for stabilising the positioning relative to the positioning surface and during the sampling itself.

There is a need to obtain an adequate stabilising effect also due to the variability in positioning of the suspicious zone relative to the positioning surface and, therefore, also due to the variability of the position of the patient relative to the positioning surface, as well as the variability in the shape and/or dimensions of the part of the body which comprises the suspicious zone.

The apparatus comprises a movement system for imparting on the probe a movement with a view to ensuring that the probe can be positioned adequately to perform the sampling. In that sense, the movement of the probe must ensure that the probe can be positioned in a correct picking up position to increase as much as possible the probability that the at least one sample picked up actually belongs to the suspicious zone.

Even though the stabilising element is functional also for the picking up, it is an object that the probe must avoid to perform the picking up. Depending on the positioning of the suspicious zone relative to the stabilising element, it might be that, due to a predetermined positioning with respect to the stabilising element of the suspicious zone, it is complicated to ensure that the probe can reach a picking up position which is sufficiently correct as mentioned above.

There is therefore the need to facilitate the picking up operation due to the variability in the positioning of the suspicious zone relative to the stabilising element.

The invention relates to an apparatus for the X-Ray analysis of samples picked up by biopsy.

An apparatus according to the invention makes it possible to achieve the aim of satisfying both these requirements.

The aim is also achieved by means of an apparatus having the features resulting from any one of, or any combination of one or more of, the appended claims.

The features of an apparatus according to the invention will become clearer from the detailed description below relating to a possible embodiment of an apparatus according to the description, given by way of non-limiting examples of the concepts claimed.

The following detailed description refers to the accompanying drawings, in which:
- Figure 1 is a perspective view of the above-mentioned possible embodiment;

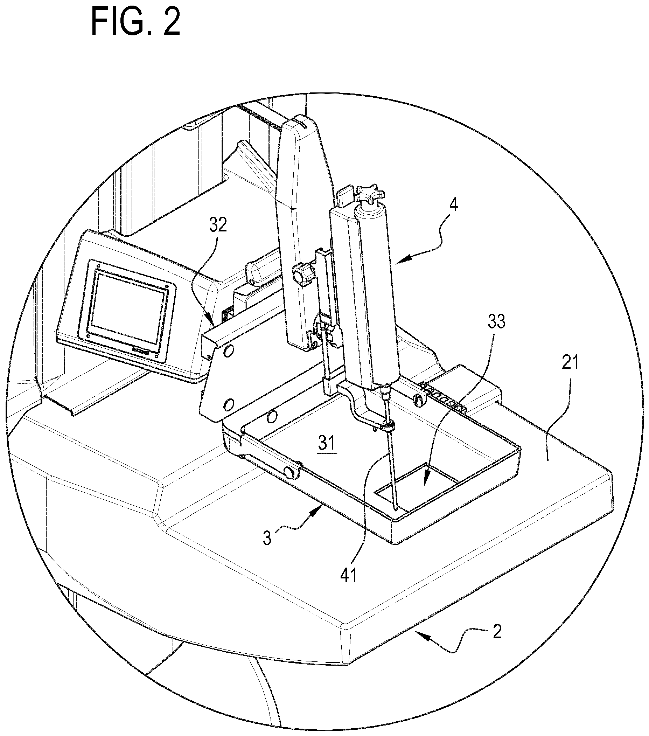

- Figure 2 is a detailed view of a part of Figure 1;
- Figures 3 and 4 illustrate a possible movement to which a component of the possible embodiment may be subjected;
- Figures 5 and 6 are, respectively, a side view and a view from above of a possible part of the body, whilst the part of the body adopts an operating position relative to the embodiment.

The accompanying drawings refer to a possible embodiment of an apparatus according to the invention.

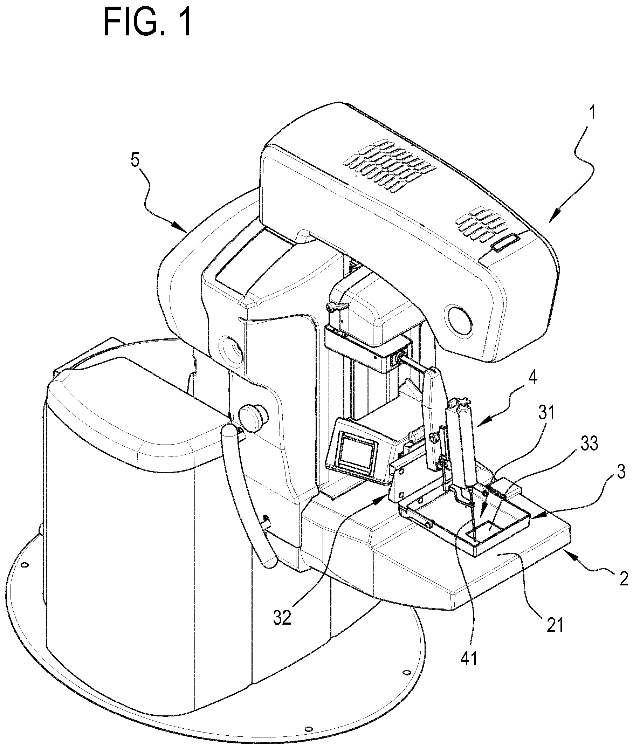

The possible embodiment is labelled 1 in Figure 1. The term " apparatus" is used below with reference to the above-mentioned possible embodiment of the apparatus.

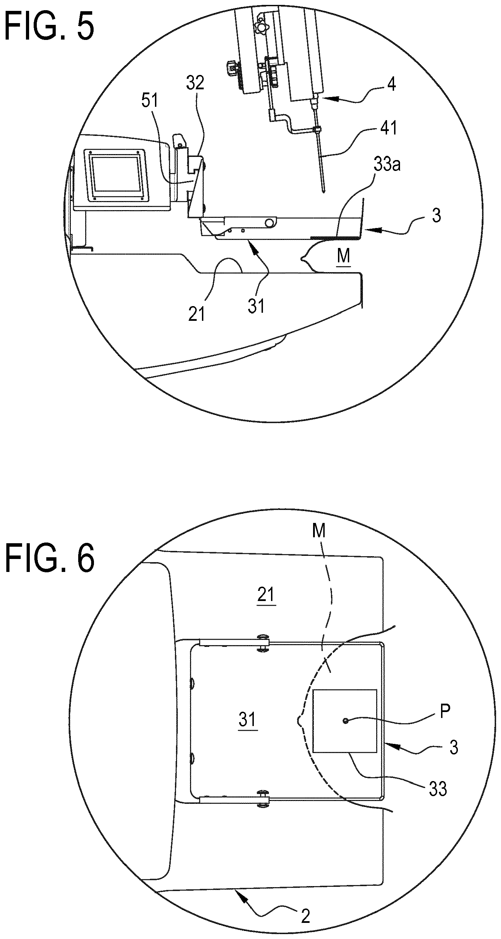

The apparatus 1 is aimed and/or configured for X-Ray analysis of samples picked up from a part of the body of a patient. The part of the body might be, for example, a breast, labelled M in Figures 5 and 6.

The apparatus 1 comprises a support 2. The support 2 is on the positioning surface 21. The support comprises an X-Ray detector.

The apparatus 1 is configured so that the part M of the body can adopt an operating position on the positioning surface 21. The operating position may be considered with respect to the positioning surface 21. In Figures 5 and 6 the part M adopts a possible operating position.

The apparatus 1 comprises a biopsy probe 4. The biopsy probe comprises a pick-up element 41. The pick-up element 41 may be, for example, a needle. The pick-up element 41 is designed to pick up of at least one sample from the part M, whilst the part M adopts the operating position. The apparatus 1 comprises a stabilising element 3. The apparatus 1 is configured so that stabilising element 3 can exert a stabilising action on the part M of the body, in such a way as to stabilise the operating position of the part M of the body relative to the positioning surface 21.

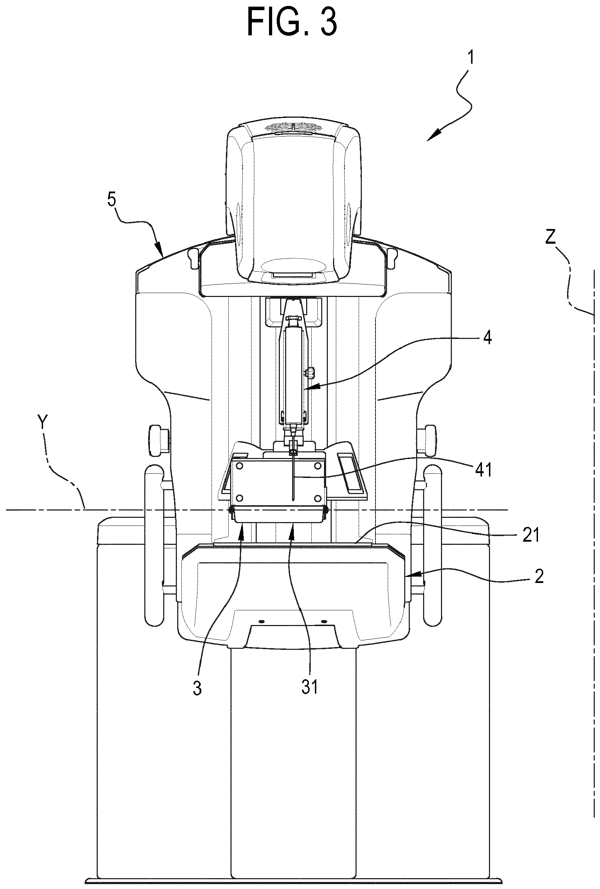

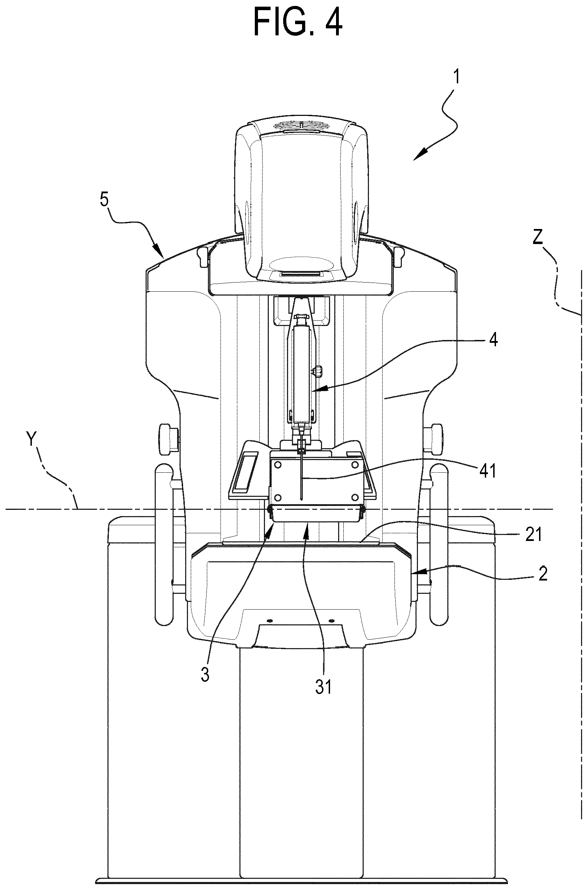

The apparatus 1 is configured for guiding at least a first movement of the stabilising element 3 relative to the positioning surface 21. The first movement of the stabilising element 3 is away from/towards the positioning surface 21. The first movement comprises at least one translational component along a first direction Z. The first direction Z is transversal and/or at right angles to the positioning surface 21. In Figures 3 and 4 the first direction Z is represented by an axis parallel to the first direction Z.

The apparatus 1 is configured for guiding at least a second movement of the stabilising element 3 relative to the positioning surface 21. The second movement of the stabilising element 3 comprises at least one translational component along a second direction Y parallel to the positioning surface 21.

The apparatus is configured for guiding the first movement and second movement independently from one another.

The first movement and second movement are aimed at least at adjusting the position of the stabilising element 3 as a function of the operating position of the part M of the body and/or its shape and/or its size.

The apparatus 1 comprises a movement system for imparting the first movement and/or the second movement.

The movement system could comprise one or more motors. For this reason, the apparatus 1 might be configured to impart the first movement and/or the second movement in a motor-driven fashion.

The apparatus 1 could comprise at least one control system by which a user can act on the first movement system and/or on the second movement system, in such a way as to cause and/or control, independently from one another, the first movement and/or the second movement.

It should be noted that the apparatus 1 could also be configured so that a user can cause and/or control the first movement and/or the second movement manually. For this reason, the apparatus 1 could be configured so that a user can cause and/or control manually one of either the first movement and second movement, and the other one using the control system, or both manually or both using the control system.

In Figures 3 and 4 stabilising element 3 is, respectively, in two different positions along the second direction Y.

The apparatus 1 comprises a window 33. The window 33 is defined through the stabilising element 3. The stabilising element 3 comprises the window 33.

The stabilising element 3 comprises a panel 31 which comes into contact with the part M of the body, to apply the above-mentioned stabilising action. The window 33 is defined through the panel 31. The panel 31 comprises the window 33. The panel 31 extends along a surface transversal and/or at right angles to the first direction Z.

The apparatus 1 is configured so that the window 33 leaves uncovered at least one sector S of the outer surface of the part M of the body, whilst the part M adopts the above-mentioned operating position. The picking up of the at least one sample by the pick-up element 41 may be performed by inserting the pick-up element 41 through the sector S, which in turn, as mentioned above, is left uncovered by the window 33. In Figure 5 the thick line labelled 33a is the projection on the plane of the drawing of an edge of the window 33. In Figure 6 the reference character P denotes a possible pick-up point, through which the pick-up element 41 can enter in the part M to perform the picking up. The presence of the window 33, leaving uncovered the sector S, makes it possible to extend in its entirety the uncovered portion of the outer surface of the part M of the body, in such a way as to make it easier for the probe 4 to perform the picking up, with the variability in the positioning of the suspicious lesion. The sector S is indicated in Figure 6, in which the window 33 is indicated on the edge corresponding to the edge 33a of Figure 5.

The stabilising element 3 comprises a sliding body 32, which supports the panel 31. The panel 31 is fixed relative to the sliding body 32. The apparatus comprises a frame 5, shown in Figures 1, 3 and 4. The frame 5 comprises a guide 51. The stabilising element 3 is mounted in the apparatus 1 in such a way that the sliding body 32 is slidable along the guide 51. The translation component of the movement of the stabilising element 3, along the above-mentioned second direction Y, is guided by the sliding of the body 32 on the guide 51. The guide 51 is indicated in Figure 5.

It should be noted that the stabilising element 3 also operates during the steps for finding and pointing, during which, by capturing X-Ray images, the suspicious zone is found and, respectively, pointed to. During these steps for finding and pointing, the stabilising action exerted by the panel 31 could comprise a pressure action. The pressure action is made possible thanks to the possibility for the stabilising element 3 to move along the above-mentioned first direction Z, and is also achieved by contact of the panel 31 with the part M of the body. This pressure action causes an extension of the part M of the body, along a direction parallel to the second direction Y, to increase the accuracy in collecting the X-Ray images of the most interesting internal components in diagnostic terms. A smaller panel 31 may therefore exert a more localised pressure action, in such a way that the pressure action produces an extension in particular of a predetermined portion of the part M of the body.

The stabilising element 3 is mounted in the apparatus 1 in such a way that it can be replaced by separating the sliding body 32 from the guide 51. More specifically, it should be noted that, depending on the type of stabilising element 3, the extension of the panel 31, along the second direction Y, may be different. As the extension of the panel 31 along the second direction Y reduces, the degree of localisation along the same second direction Y increases with which the panel 31 may exert the above-mentioned pressure action.

The usefulness of the mobility of the stabilising element 3 along the second direction Y therefore increases with the reduction in the extension of the panel 31 along the second direction Y, since it may be necessary to position the panel 31 in such a way that stabilising element 3 can exert the above-mentioned pressure action on a predetermined portion of the part M.

Moreover, it should be noted that, with the decrease in the size of the part M of the body along the second direction Y, a panel 31 with a smaller extension along the second direction Y could be more useful, since the surgeon might have the need to intervene manually on the part M of the body. A panel 31 which is too large along the second direction Y could be awkward for the manual intervention of the surgeon.

The stabilising element 3 illustrated in the accompanying drawings has an extension of the panel 31, along the above-mentioned second direction Y, less than the extension of the positioning surface 21 along the second direction Y.

The apparatus 1 is configured for guiding at least one movement of the probe 4 relative to the stabilising element 3, in such a way that the probe 4 can perform the picking up by inserting the pick-up element 41 through the sector S left uncovered by the window 33.

The apparatus 1 could also be configured to impart, by means of the above-mentioned movement system, the at least one movement of the probe 4.

The apparatus 1 could be configured so that the user, by means of the above-mentioned control system, can also control and/or cause the at least one movement of the probe 4, independently from the above-mentioned movements of the stabilising element 3.

It should be noted that, in any case, the apparatus 1 could also be configured so that a user can cause and/or control manually the at least one movement of the probe 4.

The at least one movement of the probe 4 could also comprise at least one translational component along the above-mentioned second direction Y.

An apparatus according to the invention makes it possible to obtain the above-mentioned advantages in terms of localisation of the stabilising action and/or convenience for the surgeon, even allowing use of a very small stabilising element 3, as well as more easily obtaining a correct picking up position of the probe 4.

## Claims

1. An apparatus (1) for X-Ray analysis of samples picked up from a part (M) of the body of a patient, comprising:
- a positioning surface (21), the part (M) of the body being able to adopt an operating position on and relative to the positioning surface (21);
- a biopsy probe (4) comprising a pick-up element (41), to pick up of at least one of the samples from the part (M) of the body which is operatively in the operating position;
- a stabilising element (3) for exerting a stabilising action on the part (M) of the body, in such a way as to stabilise the operating position of the part (M) of the body relative to the positioning surface (21);
wherein:
- the apparatus (1) is configured for guiding at least a first movement of the stabilising element (3) relative to the positioning surface (21), the first movement being for moving towards/away from the positioning surface (21) and comprising at least one translational component along a first direction (Z) at right angles to the positioning surface (21);
- the apparatus (1) is configured for guiding at least one second movement of the stabilising element (3) relative to the positioning surface (21), the second movement comprising at least one translational component along a second direction (Y) parallel to the positioning surface (21);
the first movement and second movement being aimed at adjusting the position of the stabilising element (3) as a function of the operating position and of the shape and/or the size of the part (M) of the body;
- the apparatus (1) comprises a window (33) defined through the stabilising element (3), the apparatus (1) being configured so that the window (33) leaves uncovered at least one sector (S) of the outer surface of the part (M) of the body, in such a way that the picking up can be performed by inserting the pick-up element (41) through the sector (S);
- wherein the stabilising element (3) comprises a panel (31) designed come into contact with the part (M) of the body to exert the stabilising action and a sliding body (32) slidable along a guide (51), in such a way that the translational component along the second direction (Y) is guided by the sliding of the body (32) on the guide (51), the window (33) being defined through the panel (31);
- wherein the stabilising element (3) is mounted in the apparatus (1) in such a way as to be it can be replaced by separation of the sliding body (32) from the guide (51).

2. The apparatus (1) according to claim 1, wherein the panel (31) extends on a surface transversal to the first direction (Z) and has an extension, along the second direction (Y), less than the extension of the positioning surface (21) along the second direction (Y).

3. The apparatus (1) according to claim 1 or 2, wherein the apparatus (1) is configured for guiding at least one movement of the probe (4) relative to the stabilising element (3), in such a way that the probe (4) can perform the picking up by inserting the pick-up element (41) through the sector (S).

4. The apparatus (1) according to any one of the preceding claims, comprising a support (2) which defines the positioning surface (21) and which comprises an X-Ray detector.

5. The apparatus (1) according to claim 3 or 4, wherein the movement of the probe (4) comprises at least one translational component along the second direction (Y).

6. The apparatus (1) according to any one of the preceding claims, comprising:
- a movement system for imparting at least one of the first movement and second movement of the stabilising element (3);
- a control system by which a user can act on the movement system, in such a way as to cause and/or control the at least one of the first movement and second movement of the stabilising element (3).

7. The apparatus according to claim 6, configured so that a user can cause and/or control manually another of the first and second movements.
